# EUROPEAN PATENT SPECIFICATION

(11) **EP 1 871 452 B1**
(45) Date of publication and mention of the grant of the patent: **30.11.2011**
(21) Application number: 06750804.4
(22) Date of filing: 19.04.2006
(51) Int. Cl.: A61M 25/00

(54) **SOFT-FLOW AORTIC CANNULA TIP**
SANFTFLIESSENDE AORTA-KANÜLENSPITZE
POINTE DE CANULE AORTIQUE HAUTE FLUIDITE

(30) Priority: 25.05.2005 US 138513; 21.04.2005 US 673939 P
(43) Date of publication of application: 02.01.2008
(73) Proprietor: Edwards Lifesciences Corporation, Irvine, CA 92614 (US)
(72) Inventor: SAUNDERS, Neil, South Jordan, UT 84095 (US); JACOBS, Brian, West Jordan, UT 84084 (US); BULTMAN, Jason, Salt Lake City, UT 84106 (US)
(74) Representative: Parry, Christopher Stephen
(86) International application number: PCT/US2006/014853
(87) International publication number: WO 2006/115967

(56) References cited:
- EP-A- 1 092 449
- WO-A-02/43795
- WO-A2-2004/009323
- US-A- 5 180 387
- US-A- 5 527 276
- US-A1- 2004 015 061
- US-B1- 6 592 546

## Description

### BACKGROUND OF THE INVENTION

During surgeries where the blood stream is temporarily diverted by cardiopulmonary bypass, the patient's blood is removed from the body, heated and/or oxygenated, and returned to the body through a device known as an aortic cannula. Because the blood is under pressure, efforts are being made to provide a tip for these cannula devices that introduces the blood into the aorta in a manner that minimizes trauma while maintaining a flow rate sufficient to obtain perfusion. If the stream velocity is too high, there is a risk of dislodging atheromatous or adherent thromba from the inside surfaces of the aorta, thereby producing emboli that can lead to strokes or other complications.

As the tip is to be inserted into the aorta through an incision, there is a further desire to make the tip as small as possible. However, smaller tips necessarily result in higher exit velocities that damage the blood cells.

Many efforts have been made at finding an optimal compromise between tip size and exit velocity. By changing the size, shape and location of the exit openings in the tip, the exit velocities can be varied greatly. Makers of other such devices have attempted to distinguish themselves by creating signature spray patterns. However, in order to generate a spray pattern, high velocities must be used 6234_1 RMI-5831 PCT even if they are associated with small flow volumes.
WO 2004/093923 discloses an aortic cannula which attempts to address this problem. The cannula has an elongate generally tubular side wall defining a conduit with distal and proximal ends. The distal end is adapted for insertion into the aorta during heart surgery to provide blood to the aorta. The conduit is separated into plural elongate lumens having smooth uninterrupted side walls and substantially equal cross sections extending back from the distal end of the cannula. The distal end is configured to permit substantially unrestricted laminar flow from all lumens out the distal end of the cannula.

The continuing pursuit of the optimal configuration is indicative that a need continues to exist for an aortic cannula tip that has a small profile, yet delivers adequate quantities of blood into the aorta at a non-traumatic flow rate without damaging the blood.

### BRIEF SUMMARY OF THE INVENTION

The aortic cannula tip of the present invention meets the aforementioned needs by incorporating a plurality of openings that disperse the exit velocity of fluid passing therethrough. The openings are constructed and arranged to create specific relief angles that disrupt the flow pattern and soften the peak velocity force of the blood as it exits the cannula tip.

The flow exits the tip through a plurality of openings that are shaped to create a flame-shaped flow pattern. The fluid flares slightly upon leaving the opening and then collapses upon itself resulting in average exit velocities that are lower than the flow velocities of the individual streams of the aforementioned prior art devices. The result of the configuration is a soft stream that is characterized by a lack of individual spray streams.

In one embodiment, the flow leaves the tip through five openings, one of which includes the longitudinal axis of the device. The distally tapered tip forces the maximum volume of fluid through this center opening with excess fluid flow being forced out the remaining four side openings. Approximately one third of the fluid exiting the tip passes through the center opening. The flared streams merge together after exiting the tip to create the desired soft stream.

Additionally, the aortic cannula tip of the present invention contains no exit openings on the top surface thereof. This allows the placement of a secondary component, such as a filter, on the top surface of the tip. By avoiding the placement of exit openings proximate the filter, the blood jet stream will flow away from the filter thereby preventing any emboli trapped in the filter from becoming dislodged. This configuration makes the aortic cannula tip of the present invention ideally suited for use with an intraaortic filtration system such as those shown and described in U.S. Patent Nos. 6,592,546, 6,589,264, 6,090,097, and 6,231,544. These systems are filter devices deployed via the arterial cannula to capture debris that may occur from an aortic cross clamp or manipulation of the heart during surgery.

### BRIEF DESCRIPTION OF THE DRAWINGS

Figure 1 is a perspective view of an embodiment of the device of the present invention;

Figure 2 is a side elevation of an embodiment of the device of the present invention;

Figure 3 is a sectional view of the device of Figure 1 taken along section lines 3-3;

Figure 4 is a sectional view of the device of Figure 2 taken along section lines 4-4;

Figures 5-6 are perspective views of an embodiment of the device of the present invention;

Figures 7-8 are perspective views of an embodiment of the device of the present invention creating a flame-shaped flow pattern;

Figures 9-10 are computer representations of flow patterns exiting a prior art device and the device of the present invention, respectively;

Figure 11 illustrates an embodiment of the present invention being used with an intraaortic filtration system.

### DETAILED DESCRIPTION OF THE INVENTION

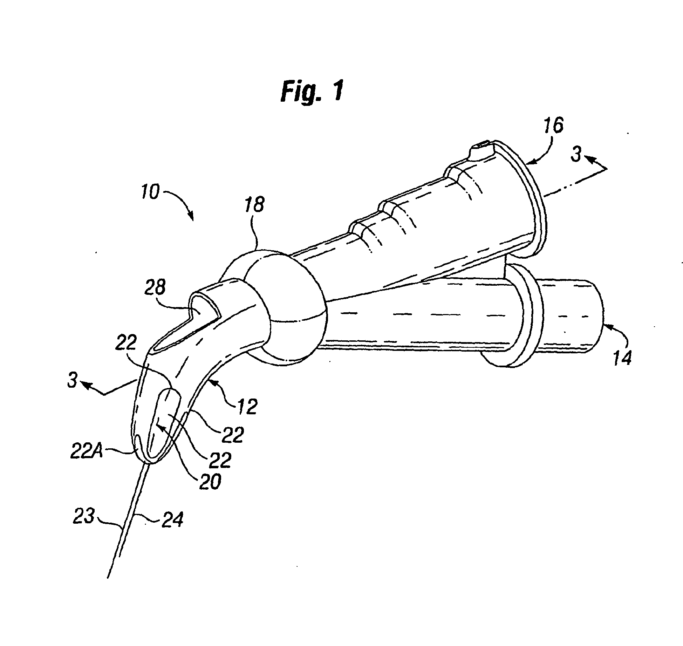

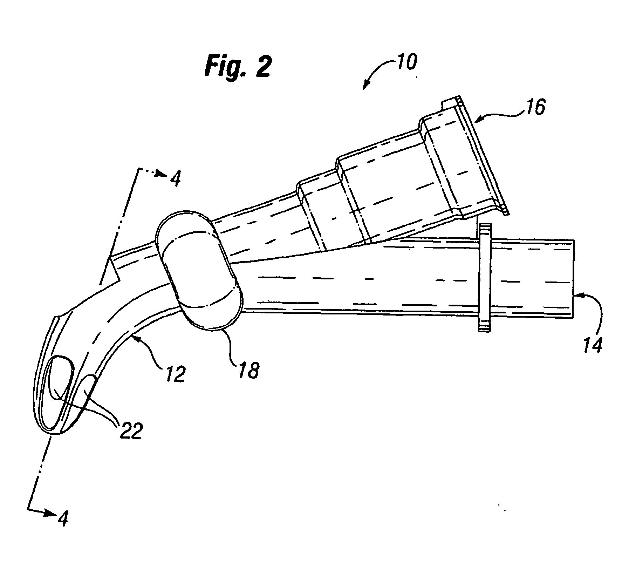

The present invention pertains to an aortic cannula tip that is attachable to a flexible cannula of a cardiopulmonary bypass circuit; a device used to supply blood back into the aorta during surgery that relies on cardiopulmonary bypass. Figures 1 and 2 show that the aortic cannula tip 10 includes a distal nozzle portion 12 extending distally from a first port 14 and a second port 16. A ridge 18 extends radially between the distal nozzle portion 12 and the ports 14 and 16.

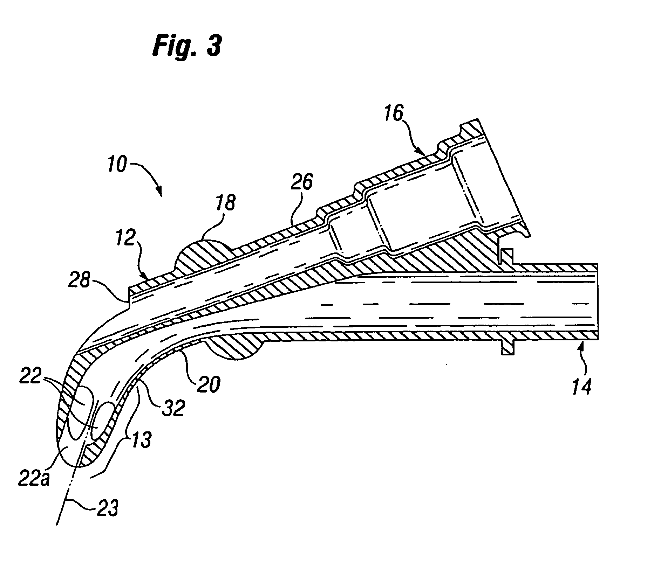

The nozzle portion 12 includes a first lumen 20 in fluid communication with the first port 14, which attaches to a flexible cannula of a cardiopulmonary bypass circuit (not shown). Figure 3 shows that the first lumen 20 is curved to follow the shape of the nozzle portion 12, which is shaped to allow a surgeon to access the aorta at an angle relatively normal to the exterior surface of the aorta. Once an incision is made in the aorta and the very distal end of the distal nozzle portion 12 has penetrated the incision, the tip 10 is rotated along the bend of the nozzle portion 12 such that the general direction of the flow exiting from the nozzle portion 12 is relatively parallel to the normal flow path through the aorta.

The nozzle portion 12 has a tapered portion 13 that tapers continually in the distal direction to the very distal tip of the nozzle portion 12. This tapered portion 13 allows the cannula tip 10 to gently and progressively open the incision in the aorta. The tapered portion 13 thus minimizes trauma and the risk of tearing. The tapered portion 13 also causes the aorta to provide continually increasing resistance as the cannula tip 10 is advanced. The embodiment of the tip 10 depicted in the Figures has a tapered portion 13 that has a 37% reduction in tip size from a proximal side of the tapered portion 13 to the very distal tip.

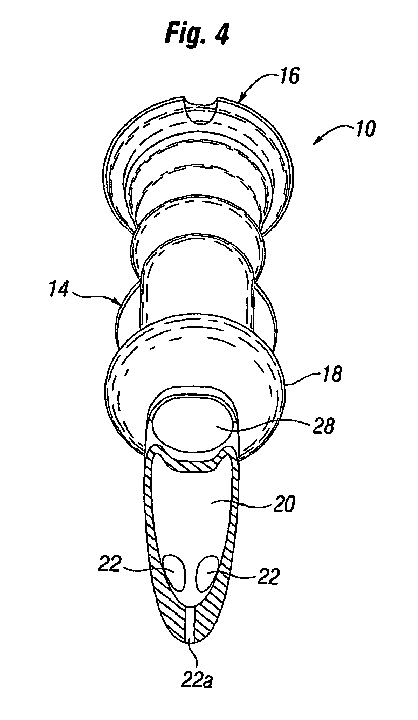

Similarly, the first lumen 20 is tapered distally in the nozzle portion 12, as best shown in Figures 3 and 4. The lumen 20 carries the blood being pumped back into the aorta. The blood exits the lumen 20 through a plurality of exit openings 22. The taper of the first lumen 20 causes the blood to accelerate gently until it reaches the openings 22. The walls 32 of the tip 10 are of relatively constant thickness in order to facilitate a tapered exterior as well as a tapered lumen 20. The tip 10 is constructed using a rigid material such that the tapers are preserved despite fluid pressures. In other words, the rigid material prevents the first lumen 20 from swelling or straightening when fluid under pressure is introduced into the lumen 20. Similarly, the thickness of the walls 32 does not change when the tip 10 is subject to fluid pressure.

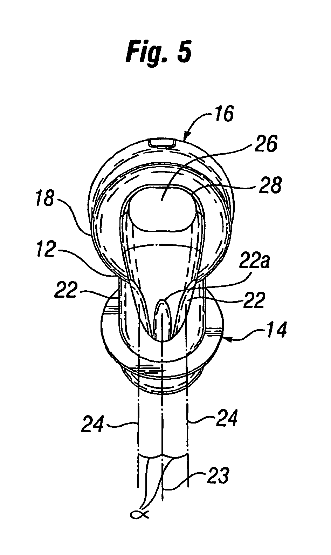

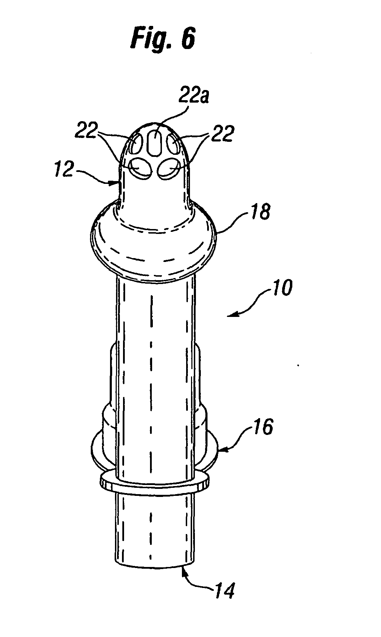

As best seen in Figures 5 and 6, the illustrated embodiment has five exit openings 22. One of the exit openings, opening 22a, wraps around the very distal end of the tip 10. This opening 22a is located such that the longitudinal axis 23 of the central lumen 20 passes through the opening 22a. As a result, more blood passes through opening 22a than the other openings 22. Preferably, approximately one third of the blood exiting the tip 10 passes through the central opening 22a. The other openings 22 each have longitudinal axes 24 that are roughly parallel to, but offset from, axis 23. Preferably, the other openings have roughly parallel longitudinal axes 24 that are splayed outwardly less than two degrees from the longitudinal axis 23. More preferably, the other openings have longitudinally axes 24 that are splayed approximately one degree from the longitudinal axis 23 of the central lumen. The angle between the longitudinal axis 23 of the central lumen and the longitudinal axis 24 of any given opening 22, may be hereinafter referred to as a "relief angle" and is shown in Figure 5 as angle α.

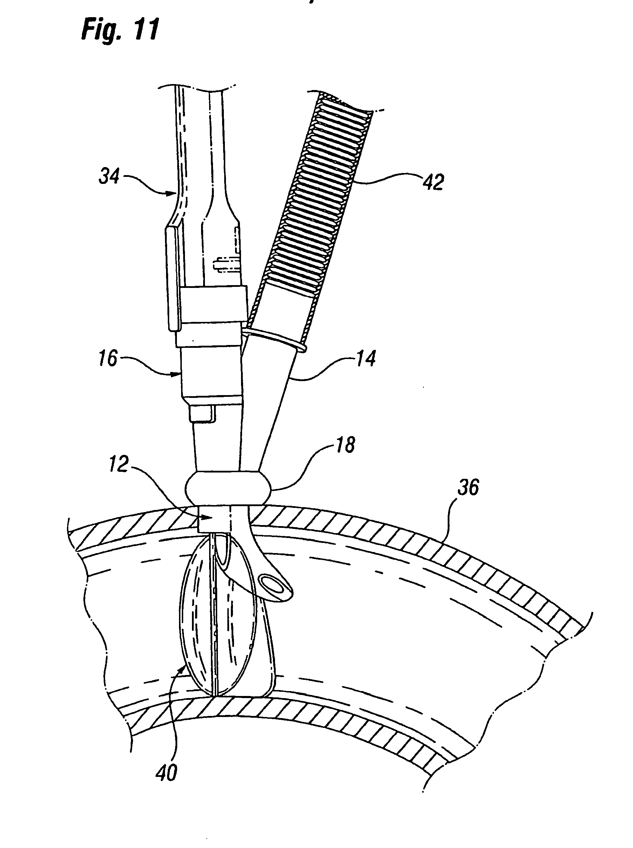

Referring back to Figure 3, the second port 16 defines a second lumen 26 that leads to an auxiliary opening 28. The opening 28 is usable for attaching a secondary component, such as a filter (Figure 11), to the tip 10. Exemplary filter devices can be found in U.S. Patent Nos. 6,592,546, 6,589,264, 6,090,097, and 6,231,544 and are discussed in more detail below. The exit opening 22a does not extend in the proximal direction as far as the other openings 22 in order to accommodate the introduction of a secondary component without interference between the secondary component and the fluid flow.

The first port 14 and the second port 16 are constructed and arranged for attachment to an extracorporeal bypass pump (Figure 11). The first port 14 and second port 16 are separated from the nozzle portion 12 by a ridge 18. The ridge 18 performs three functions. First, the ridge 18 provides feedback to the user when it contacts the arterial wall. Thus, the user feels resistance and is thus assured that the nozzle portion is fully inserted. Second, the ridge 18 forms a seal against the arterial wall to prevent leakage through the incision. Third, the ridge 18, being rounded, allows the user to change the angle of the tip without breaking the seal between the aorta and the ridge. Preferably, the first port 14, second port 16 and the ridge 18 are of unitary construction, as shown in Figure 3. Additionally, the tip 10 has a lumen wall 32 that defines the curve of the tip 10. In a preferred embodiment, this wall 32 is of substantially uniform thickness.

In operation, the first port 14 and second port 16 are attached to a flexible cannula, which is used in the extracorporeal bypass circuit. A pump in the circuit sends oxygenated blood through the first port 14 into lumen 20. The blood follows the curvature of the lumen in the nozzle portion 12, where the taper of the lumen gently accelerates the blood through the openings 22.

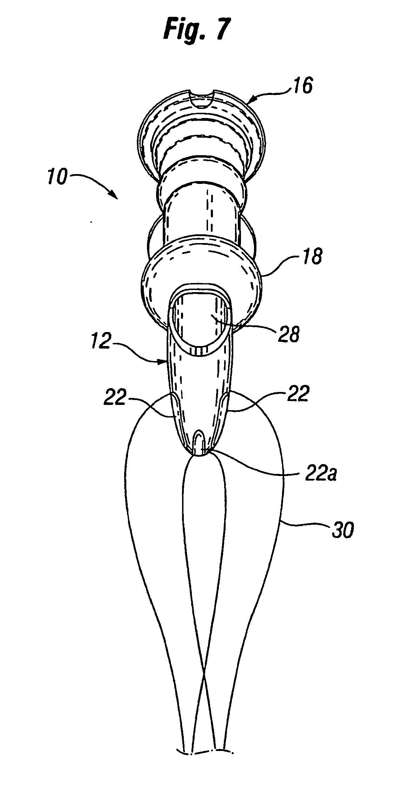

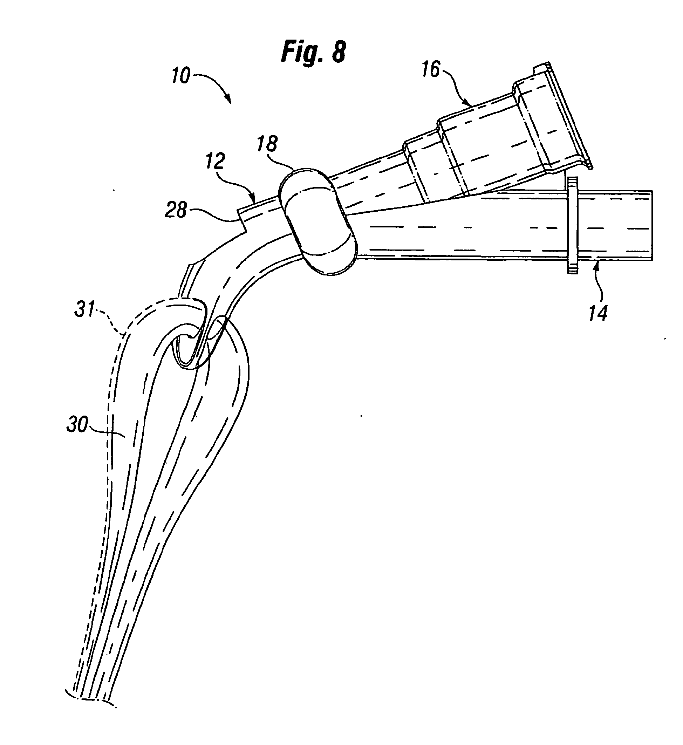

The openings 22 are constructed and arranged to create a stream 30 that is flame-shaped when sprayed in open air (Figures 7 and 8). The flame-shaped stream 30 is a result of the acceleration of fluid through the tapered lumen 20, the shape of the openings 22, and the amount of fluid passing through the central opening 22a. Approximately one third of all the fluid passing through the tip 10 passes through the central opening 22a. The accelerating fluid finds relief first at the proximal edges of the openings 22, causing fluid to flare outwardly therefrom. However, because the openings have relief angles that are very small (less than two degrees from the longitudinal axis 23 of the central lumen), the flaring creates low pressure that draws the fluid back to the longitudinal axes 24. Because approximately one third of the fluid is passing through the central opening 22a roughly along the central longitudinal axis 23, a further strong venturi draw is created toward the center axis 23, thereby tapering the stream 30. The collapsing of the flared fluid back toward the central axis 23 also causes turbulence and significantly decreases the fluid velocity peak force. The result is a softer, less traumatizing stream 30.

In actual use, the stream enters the aorta, which already has blood flowing through it. Thus, the flame-shaped stream 30 does not maintain a flame-shape due to the turbulence it creates with the surrounding blood. However, the effect the stream has on the surrounding blood helps create the softer flow. Rather than producing radiating spray that impacts the walls of the aortic lumen, the stream draws the surrounding blood into it, thereby protecting the aortic walls from direct impingement. Thus, a softer introduction of blood into the aorta is provided.

Comparing Figure 7 with Figure 8, the flame-shaped flow pattern 30 includes an area 31 (shown in phantom lines) proximate the auxiliary opening 28 lacking flow due to the shape of opening 22a. This area allows a filtration system to be deployed without interference from the flow 30.

The design of the openings 22 ensures that the velocities of the blood streams through the openings 22 do not decrease until immediately after the fluid has left the aortic cannula tip 10. Thus, the exit velocities through the openings are high enough to prevent clotting. The tip 10 achieves both reduced fluid velocities in the aorta and increased flow velocities through the openings, where clotting is to be avoided.

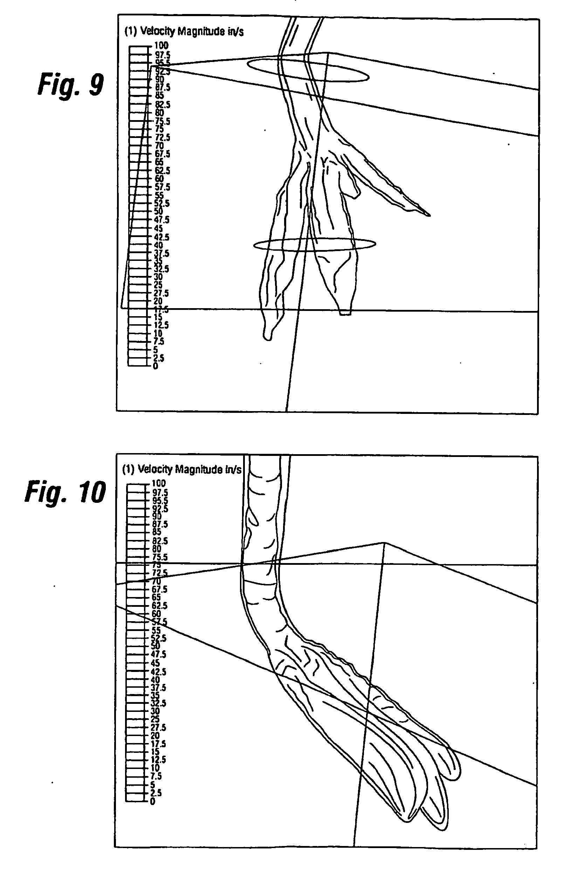

Referring to Figures 9 and 10, the effect created by the small relief angles of the openings 22 is further illustrated. Figure 9 is a computer-generated representation of the flow pattern 50 of a tip having relief angles greater than those of the present invention. The actual tip being omitted from the Figure, the flow 50 begins as a single solid stream 52, traveling at 0,927 m/second (36.4 inches/second), and contained within the central lumen of the device. The flow 50 then splays into individual branches 54 as it passes through openings having large relief angles, which direct the flow outwardly. Introduced into an aorta, these branches 54 would impinge on the aortic walls. Figure 10, on the other hand, is a computer-generated representation of the flow pattern 60 of a tip 10 of the present invention, having relief angles of approximately one degree. Again, the tip is omitted from the Figure, which begins with a solid stream 62, also traveling at 0.927 m/second (36.4 inches/second), passing through the central lumen of the device. The stream bulges at 64 where the flow exits the openings 22. However, the small relief angles, and the other aforementioned fluid dynamics created thereby, prevent individual streams from forming. Rather the stream collapses upon itself and maintains the characteristics of a single, soft stream 66. When the stream collapses upon itself, it draws native fluid inward and mixes with the introduced stream 66.

Figure 11 illustrates the aortic tip 10 being used with an intraaortic filtration system 34, such as the systems discussed in U.S. Patent Nos. 6,592,546, 6,589,264, 6,090,097, and 6,231,544. In Figure 11, the aortic tip 10 has been inserted into an aorta 36 in order to deploy the filtration system 34. The rounded ridge 18 maintains an effective seal against the aorta despite being pivoted. The filtration system includes an introducer 34 and a filter assembly 40. The introducer passes through the second port 16 into the second lumen 26 and out the auxiliary opening 28. Due to the absence of openings 22 proximate the auxiliary opening 28, no interference occurs between the flame-shaped flow 30 and the filter assembly 40. Also shown is a flexible cannula 42 attached to the first port 14.

Although the invention has been described in terms of particular embodiments and applications, one of ordinary skill in the art, in light of this teaching, can generate additional embodiments and modifications without departing or exceeding the scope of the claimed invention. Accordingly, it is to be understood that the drawings and descriptions herein are proffered by way of example to facilitate comprehension of the invention and should not be construed to limit the scope thereof.

## Claims

1. An aortic cannula comprising:
a body (10) defining a central lumen (20) having a central axis (23); and
a curved distal nozzle portion (12) having a tapered tip (13) with a central opening (22a) in line with the central axis at the tip of the nozzle and in fluid communication with the central lumen (20), which lumen tapers distally and
a plurality of further openings (22) in fluid communication with the central lumen (20) arranged around the central opening (22a), each of the further openings (22) having a longitudinal axis (24) which is substantially parallel but offset to the central axis at the tip of the nozzle.

2. The cannula of Claim 1, further comprising:
a proximal port (14) opposite said nozzle portion (12), attachable to a fluid supply such that the fluid supply communicates fluid to the central lumen.

3. The cannula of Claim 1, further comprising a second proximal port (16), opposite said nozzle portion, having a second lumen (26) and attachable to an auxiliary device.

4. The cannula of Claim 3, wherein the second lumen (26) has a distal opening (28) and wherein the distal opening of the second lumen and the openings (22, 22a) in the nozzle portion (12) are constructed and arranged such that a fluid flow pattern created by the nozzle openings is spaced apart from the second lumen opening (28).

5. The cannula of Claim 1, further comprising a ridge (18) radiating outwardly from the body proximal of the distal nozzle portion.

6. The cannula of Claim 1, wherein the openings (22a, 22) are shaped such that fluid exiting the openings creates a flame-shaped pattern.

7. The cannula of Claim 1, wherein the body further includes walls (32) that form the lumen, the walls being rigid and of substantially uniform thickness.

8. The cannula of Claim 1, wherein the plurality of further parallel openings (22) have relief angles between the central axis (23) and the longitudinal axis of an opening (22) of less than two degrees.

9. The cannula of Claim 2, wherein the plurality of further parallel openings (22) have relief angles of approximately one degree.

## Patentansprüche

1. Eine Aortenkanüle, die umfasst:
einen Körper (10), der ein zentrales Lumen (20) mit einer Mittelachse (23) bestimmt; und
einen gebogenen distalen Düsenabschnitt (12) mit einer konischen Spitze (13) mit einer zentralen Öffnung (22a), die mit der Mittelachse ausgerichtet ist, an der Spitze der Düse und in flüssiger Verbindung mit dem zentralen Lumen (20) steht, dessen Lumen sich distal verjüngt;
eine Vielzahl von weiteren Öffnungen (22) in flüssiger Verbindung mit dem zentralen Lumen (20), die um die zentrale Öffnung (22a) herum angeordnet sind, wobei jede der weiteren Öffnungen (22) eine Längsachse (24) aufweist, die im Wesentlichen parallel, aber versetzt zu der Mittelachse an der Spitze der Düse verläuft.

2. Die Kanüle gemäß Anspruch 1, die weiterhin umfasst:
einen proximalen Anschluss (14) gegenüber dem Düsenabschnitt (12), der so an eine Flüssigkeitszufuhrquelle anschließbar ist, dass die Flüssigkeitszufuhrquelle Flüssigkeit zu dem zentralen Lumen übermittelt.

3. Die Kanüle gemäß Anspruch 1, die weiterhin einen zweiten proximalen Anschluss (16) gegenüber dem Düsenabschnitt umfasst, der ein zweites Lumen (26) aufweist und an einer Hilfsvorrichtung anschließbar ist.

4. Die Kanüle gemäß Anspruch 3, wobei das zweite Lumen (26) eine distale Öffnung (28) aufweist und wobei die distale Öffnung des zweiten Lumens und die Öffnungen (22, 22a) in dem Düsenabschnitt (12) so konstruiert und angeordnet sind, dass ein von den Düsenöffnungen geschaffenes Flüssigkeitsflussmuster im Abstand von der zweiten Lumenöffnung (28) angeordnet ist.

5. Die Kanüle gemäß Anspruch 1, die weiterhin einen Grat (18) umfasst, der von dem Körper proximal zu dem distalen Düsenabschnitt nach außen ausstrahlt.

6. Die Kanüle gemäß Anspruch 1, wobei die Öffnungen (22a, 22) so geformt sind, dass die aus den Öffnungen austretende Flüssigkeit ein flammenförmiges Muster schafft.

7. Die Kanüle gemäß Anspruch 1, wobei der Körper weiterhin Wände (32) umfasst, die das Lumen bilden, wobei die Wände starr und von im Wesentlichen gleichförmiger Dicke sind.

8. Die Kanüle gemäß Anspruch 1, wobei die Vielzahl von weiteren parallelen Öffnungen (22) Freistellwinkel zwischen der Mittelachse (23) und der Längsachse einer Öffnung (22) von weniger als zwei Grad aufweisen.

9. Die Kanüle gemäß Anspruch 2, wobei die Vielzahl von weiteren parallelen Öffnungen (22) Freistellwinkel von ungefähr einem Grad aufweisen.

## Revendications

1. Canule aortique comprenant :
un corps (10) définissant une lumière centrale (20) ayant un axe central (23); et
une partie de bec distal courbé (12) ayant une pointe effilée (13) avec une ouverture centrale (22a) alignée sur l'axe central, à la pointe du bec et en communication fluide avec la lumière centrale (20), laquelle lumière s'amincit dans le plan distal et
une pluralité d'ouvertures supplémentaires (22) en communication fluide avec l'ouverture centrale (20) arrangées autour de l'ouverture centrale (22a), chacune des ouvertures supplémentaires (22) ayant un axe longitudinal (24) qui est sensiblement parallèle à, mais décalé de l'axe central à la pointe du bec.

2. Canule selon la revendication 1, comprenant en outre :
un orifice proximal (14) à l'opposé de ladite partie de bec (12), pouvant être attaché à une alimentation de fluide de sorte que l'alimentation de fluide communique le fluide à la lumière centrale.

3. Canule selon la revendication 1, comprenant en outre un deuxième orifice proximal (16), à l'opposé de ladite partie de bec, ayant une deuxième lumière (26) et pouvant être attaché à un dispositif auxiliaire.

4. Canule selon la revendication 3, dans laquelle la deuxième lumière (26) a une ouverture distale (28) et dans laquelle l'ouverture distale de la deuxième lumière et les ouvertures (22, 22a) dans la partie de bec (12) sont construites et arrangées de sorte qu'un diagramme d'écoulement de fluide créé par les ouvertures du bec, est espacé de l'ouverture (28) de la deuxième lumière.

5. Canule selon la revendication 1, comprenant en outre une arête (18) s'arrondissant vers l'extérieur du corps proximal de la partie distale du bec.

6. Canule selon la revendication 1, dans laquelle les ouvertures (22a, 22) sont façonnées de telle sorte que le fluide sortant des ouvertures crée un diagramme en forme de flamme.

7. Canule selon la revendication 1, dans laquelle le corps comprend en outre des parois (32) qui forment la lumière, les parois étant rigides et d'une épaisseur sensiblement uniforme.

8. Canule selon la revendication 1, dans laquelle la pluralité d'ouvertures parallèles supplémentaires (22) ont des angles de dépouille entre l'axe central (23) et l'axe longitudinal d'une ouverture (22) de moins de deux degrés.

9. Canule selon la revendication 2, dans laquelle la pluralité d'ouvertures parallèles supplémentaires (22) ont des angles de dépouille d'environ un degré.
